**Europäisches Patentamt**

**European Patent Office**

**Office européen des brevets**

(19)

(11) Veröffentlichungsnummer : **0 365 825 B1**

(12) ## EUROPÄISCHE PATENTSCHRIFT

(45) Veröffentlichungstag der Patentschrift :
**01.04.92 Patentblatt 92/14**

(51) Int. Cl.$^5$ : **A61K 7/00**, A61K 7/06, A61K 7/50

(21) Anmeldenummer : **89117445.0**

(22) Anmeldetag : **21.09.89**

(54) Konservierte Haar- und Körperbehandlungsmittel sowie Verwendung einer Konservierungsstoff-Kombination.

Die Akte enthält technische Angaben, die nach dem Eingang der Anmeldung eingereicht wurden und die nicht in dieser Patentschrift enthalten sind.

(30) Priorität : **25.10.88 DE 3836241**

(43) Veröffentlichungstag der Anmeldung :
**02.05.90 Patentblatt 90/18**

(45) Bekanntmachung des Hinweises auf die Patenterteilung :
**01.04.92 Patentblatt 92/14**

(84) Benannte Vertragsstaaten :
**AT BE CH DE ES FR GB GR IT LI NL SE**

(56) Entgegenhaltungen :
**EP-A- 0 273 218**
**EP-A- 0 287 805**
**DE-A- 3 434 885**
**US-A- 4 294 852**

(56) Entgegenhaltungen :
**SOAP, PERFUMERY & COSMETICS, Band 37, Nr. 11, November 1964, Seiten 981-1014, United Trade Press, London, GB; E. LÜCK:**
**"Sorbic acid for the preservation of cosmetic preparations"**
**JOURNAL OF THE AMERICAN PHARMACEUTICAL ASSOCIATION, scientific edition, Band 46, Nr. 7, Juli 1957, Seiten 445-451, American Pharmaceutical Association, Washington, US; M. BARR et al.: "The preservation of aqueous preparations containing nonionic surfactants II"**
**SEIFEN-ÖLE-FETTE-WACHSE, Band 98, Nr. 4, 17. Februar 1972, Seiten 83-85, Verlag für Chemische Industrie, Augsberg, DE; O. PAULI: "Konservierungsmittel für Kosmetika"**

(73) Patentinhaber : **Wella Aktiengesellschaft**
**Berliner Allee 65**
**W-6100 Darmstadt (DE)**

(72) Erfinder : **Lang, Günther, Dr.**
**Auf der Roten Erde 10 B**
**W-6107 Reinheim 5 (DE)**
Erfinder : **Schwarz, Horst**
**Fliederweg 22 D**
**W-6104 Seeheim (DE)**
Erfinder : **Hölzel, Hans**
**Jahnstrasse 9**
**W-6101 Fränkisch-Crumbach (DE)**

**Beschreibung**

Haar- und Körperbehandlungsmittel enthalten in der Regel einen Konservierungsstoff, welcher die Mittel vor dem Befall durch Mikroorganismen wirksam schützen soll.

Für den Einsatz in Haar- und Körperbehandlungsmitteln werden an einen Konservierungsstoff gegensätzliche Anforderungen gestellt. So sollte er einerseits in physiologischer und dermatologischer Hinsicht gut verträglich sein und andererseits über eine gute keimhemmende oder sogar keimtötende Wirkung verfügen. Beide Anforderungen sind meist nur schwer miteinander vereinbar.

Als übliche in Haar- und Körperbehandlungsmitteln verwendete Konservierungsstoffe seien beispielsweise Formaldehyd (a), 5-Brom-5-nitro-1,3-dioxan (b), p-Hydroxybenzoesäureester (c), 2,4,4′-Trichlor-2′-hydroxy-diphenylether (d), 5-Chlor-2-methyl-3-isothiazolon (e), 2-Methyl-3-isothiazolon (f) und 2-Brom-2-nitropropan-1,3-diol (g) genannt.

In letzter Zeit sind einige Konservierungsstoffe, wie beispielsweise Formaldehyd und 2-Methyl-3-isothiazolon, in den Verdacht geraten, nicht genügend verträglich zu sein. Von konservierend wirkenden Aldehyden und Phenolen ist bekannt, daß diese mit Proteinen reagieren oder mit diesen in denaturierender Weise in Wechselwirkung treten. Bei Aldehyden besteht zudem die Gefahr der Sensibilisierung.

Ein weiteres Risiko, welches gegen eine Verwendung derartiger Konservierungsmittel spricht, ist die Gefahr der Nitrosaminbildung. Bei Rohstoffen, welche Nitrogruppen enthalten ((b) + (g)) ist dieses Risiko besonders groß, wenn sie zusammen mit anderen stickstoffhaltigen Komponenten des kosmetischen Mittels eingesetzt werden.

Es wurden bereits Konservierungsstoffe vorgeschlagen, welche die oben genannten Nachteile nicht aufweisen.

So ist in der DE-OS 3 644 473 ein Haar- und Körperreinigungsmittel beschrieben, welches ein physiologisch verträgliches Salz der Ameisensäure, Salicylsäure oder deren Salz und eine physiologisch unbedenkliche organische oder anorganische Säure enthält.

Der Einsatz von Salicylsäure als Konservierungsstoff ist jedoch nicht völlig zufriedenstellend, da die Salicylsäure bei sehr empfindlichen Menschen zu Hautausschlägen oder in Ausnahmefällen zu Asthmaanfällen führen kann. Außerdem wirkt sie beim Einsatz höherer Konzentrationen keratolytisch, was bedeutet, daß sie die Hornschicht der Haut auflösen kann.

Es bestand daher die Aufgabe, ein Haar- und Körperbehandlungsmittel mit einem Gehalt an einer Konservierungsstoff-Kombination zur Verfügung zu stellen, welche eine gute konservierende Wirkung besitzt, eine bessere physiologische Verträglichkeit aufweist als übliche in derartigen Mitteln verwendete Konservierungsstoffe sowie die vorstehend geschilderten Nachteile der Salicylsäure vermeidet.

Hierzu wurde gefunden, daß ein wäßriges Haar- und Körperbehandlungsmittel mit einem Gehalt an 0,1 bis 50 Gewichtsprozent eines Tensids oder eines Tensidgemisches, dadurch gekennzeichnet, daß es eine Kombination aus

(A) einem physiologisch verträglichen Salz der Ameisensäure,

(B) Sorbinsäure und/oder deren physiologisch verträglichem Salz und

(C) einer oder mehrerer physiologisch verträglicher anorganischer oder gesättigter aliphatischer organischer Säuren

enthält, die gestellte Aufgabe in hervorragender Weise löst.

In der Natur kommt die Sorbinsäure in der Vogelbeere (Sorbus aucuparia) vor.

Das erfindunsgemäße Mittel wird durch die vorstehend beschriebene Kombination von Konservierungsstoffen besser konserviert als das in der DE-OS 3 644 473 beschriebene Haar- und Körperreinigungsmittel mit einem Gehalt an Salicylsäure.

Aus diesem Grund kann die Einsatzkonzentration des neuen Konservierungssystems aus den Komponenten (A) - (C) deutlich niedriger gewählt werden, als dies in der DE-OS 3 644 473 der Fall ist, wodurch gleichzeitig eine bessere Verträglichkeit erreicht wird.

Während beispielsweise ein Haar- und Körperbehandlungsmittel nach der DE-OS 3 644 473, welches 0,05 Gewichtsprozent Natriumformiat und 0,05 Gewichtsprozent Salicylsäure enthält, nicht mehr ausreichend konserviert ist, übertrifft das gleiche, mit 0,05 Gewichtsprozent Natriumformiat und 0,05 Gewichtsprozent Sorbinsäure konservierte Mittel die Anforderungen des Konservierungstests.

Dies ist insbesondere deswegen überraschend, weil die Sorbinsäure alleine in niedriger Konzentration kosmetische Mittel nicht ausreichend konserviert, in höherer Konzentration jedoch, beispielsweise nach H. Janistyn, Handbuch der Kosmetika und Riechstoffe, I. Band, Seiten 396 - 397, Dr. A. Hüthig Verlag, Heidelberg (1975), als hautreizend gilt.

Die Güte der Konservierung der vorstehend beschriebenen Haar- und Körperbehandlungsmittel wurde durch den in der Literatur The United States Pharmacopeia, 21. Auflage, (1985), Seite 1151 beschriebenen Konservierungsbelastungstest festgestellt. Hierbei wurden die Haarbehandlungsmittel mit den Mikroorganismen Candida albicans (ATCC Nr. 10231), Aspergillus niger (ATCC Nr. 16404). Escherichia coli (ATCC Nr. 8739), Pseudomonas aeruginosa (ATCC Nr. 9027) und Staphylococcus aureus (ATCC Nr. 6538) verunreinigt und die Entwicklung der Keimzahl über einen Zeitraum von 28 Tragen überwacht.

Während die alleinige Verwendung von Natriumformiat oder Sorbinsäure, insbesondere bei Befall durch Pilze, wie zum Beispiel Aspergillus niger, nicht zu einer zufriedenstellenden Konservierung der Haar- und Körperbehandlungsmittel führt, werden die Mittel durch die Verwendung der vorstehend beschriebenen Kombination aus den Konservierungsstoffen (A), (B) und (C) auch gegen Pilze sehr gut konserviert.

Als geeignete physiologisch verträgliche Salze der Ameisensäure (A) seien beispielsweise das Natriumformiat, das Ammoniumformiat, das Kaliumformiat und das Magnesiumformiat genannt, wobei das Natriumformiat bevorzugt ist.

In dem Haar- und Körperbehandlungsmittel ist die Komponente (A) in einer Menge von etwa 0,01 bis 2,0 Gewichtsprozent, vorzugsweise 0,1 bis 1,0 Gewichtsprozent, enthalten.

Die Komponente (B) ist in einer Menge von etwa 0,05 bis 2,0 Gewichtsprozent, vorzugsweise 0,1 bis 1,0 Gewichtsprozent, in dem erfindungsgemäßen Mittel enthalten, wobei als Salz der Sorbinsäure vor allem ein Alkalisorbat, insbesondere das Natriumsorbat geeignet ist.

Als physiologisch unbedenkliche gesättigte aliphatische organische Säure der Komponente (C) kommen vorzugsweise von Amino-oder Halogensubstituenten freie gesättigte aliphatische organische Säuren mit 2 bis 6 Kohlenstoffatomen, wie zum Beispiel Zitronensäure, Weinsäure, Milchsäure, Adipinsäure, Glyoxylsäure, Gluconsäure und Äpfelsäure in Betracht, wobei Zitronensäure bevorzugt ist, während als anorganische Säure der Komponente (C) vorzugsweise Phosphorsäure verwendet wird.

Die Komponente (C) ist in den neuen Mitteln in einer Menge von etwa 0,1 bis 1,5 Gewichtsprozent, vorzugsweise 0,25 bis 0,5 Gewichtsprozent, enthalten.

Mit der Komponente (C) wird der pH-Wert des Haar- und Körperbehandlungsmittels eingestellt, welcher 2,0 bis 7,0, vorzugsweise 3,0 bis 5,8, beträgt.

Das erfindungsgemäße Haar- und Körperbehandlungsmittel liegt vorzugsweise in Form einer wäßrigen Lösung oder Emulsion, beispielsweise als Lotion, Gel, Creme oder Aerosol, vor und enthält neben der Kombination aus (A), (B) und (C) mindestens ein anionisches, kationisches, nicht-ionogenes oder amphoteres Tensid in einer Menge von etwa 0,1 bis 50 Gewichtsprozent, vorzugsweise 10 bis 25 Gewichtsprozent.

Von den für das neue Haar- und Körperbehandlungsmittel geeigneten Tensiden seien beispielsweise die folgenden genannt:

beispielsweise die Alkali-, Erdalkali-, Ammonium- oder Alkanolaminsalze von Alkansulfonaten, Alkylsulfonaten und Alkylethersulfaten, die $C_{12}$ bis $C_{18}$-Alkyl- und insbesondere $C_{12}$ bis $C_{14}$-Alkyl-Sulfatnatriumsalze oder -Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl- und Stearylalkohol, allein oder im Gemisch; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispeilsweise Lauryl- oder Cetylpyridiniumchlorid, die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agenzien wie beispielsweise die Carboxylderivate von Imidazol, die N-Alkylbetaine, die N-Alkylaminobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethylcarboxymethylenpropylenamido-stearat-betain.

Selbstverständlich kann das erfindungsgemäße Mittel neben den genannten Bestandteilen auch übliche kosmetische Zusätze, beispielsweise parfümöle in einer Menge von etwa 0,5 bis 5,0 Gewichtsprozent, Trübungsmittel, wie zum Beispiel Ethylenglykoldistearat, in einer Menge von etwa 0,5 bis 5,0 Gewichtsprozent, Perlglanzmittel, wie zum Beispiel ein Gemisch aus Fettsäuremonoalkylolamid und Ethylenglykoldistearat, in einer Menge von etwa 1,0 bis 10,0 Gewichtsprozent, Verdickungsmittel, wie beispiels weise Kokosfettsäurediethanolamid, in einer Menge von etwa 0,5 bis 10,0 Gewichtsprozent, Verdünnungsmittel, wie zum Beispiel 1,2-Propylenglykol, niedere aliphatische Alkohole oder ethoxyliertes Sorbitanmonolaurat, in einer Menge von etwa 0,5 bis 5,0 Gewichtsprozent, Puffersubstanzen, wie beispielsweise Natriumcitrat oder Natriumphosphat, in einer Menge von etwa 0,1 bis 1,0 Gewichtsprozent, Lösungsvermittler, wie zum Beispiel ethoxyliertes, gegebenenfalls hydriertes Rizinusöl, in einer Menge von etwa 0,1 bis 1,0 Gewichtsprozent, sowie Farbstoffe, wie zum Beispiel Fluorescein-Natriumsalz, in einer Menge von etwa 0,1 bis 1,0 Gewichtsprozent, weiterhin haar- und hautpflegende Zusätze, wie zum Beispiel Fettsäureester, Fettalkohole, Fettsäureglyceride, ethoxylierte oder propoxylierte gesättigte Fettalkohole, natürliche, modifizierte natürliche oder synthetische Polymere, wie zum Beispiel Cellulosederivate, kationische Cellulosederivate, Chitosan, kationische Chitinderivate oder Polymerisate von Acrylsäure und/oder deren Derivaten, Pflegestoffe, wie zum Beispiel Lanolinderivate, Cholesterin und Pantothensäure in einer Menge von etwa 0,1 bis 10 Gewichtsprozent, außerdem physiologisch verträgliche anorganische Salze, wie zum Beispiel Natriumchlorid, sowie ferner Feuchthaltemittel, Farbstoffe, Lichtschutzmittel, Antioxidantien, Komplexbildner und Antischuppenwirkstoffe enthalten.

Weitere übliche, für derartige Mittel bekannte Bestandteile, welche in dem neuen Mittel enthalten sein können, sind beispielsweise bei H. Janistyn, "Handbuch der Kosmetika und Riechstoffe", 3. Band, (1973), Seiten 228 - 284 und 442 - 462, K. Schrader, "Grundlagen und Rezepturen der Kosmetika", (1979), Seiten 375 - 401 und 445 - 455 sowie G. A. Nowak, "Die kosmetischen Präparate", (1984), Seiten 452 - 512, beschrieben.

Die nachfolgenden Beispiele sollen den Gegenstand der Erfindung näher erläutern.

## HAAR- UND KÖRPERBEHANDLUNGSMITTEL

### Beispeil 1: Haarwaschmittel

```
0,1 g      Sorbinsäure

0,1 g      Natriumformiat

40,0 g     Laurylalkohol-diglykolethersulfat,

           Natriumsalz (28 prozentige, wäßrige Lösung)

0,5 g      Parfümöl


4,5 g      Natriumchlorid

54,7 g     Wasser

100,0 g
```

Der pH-Wert wird mit verdünnter Natronlauge auf 4,8 - 5,8 eingestellt. Das vorstehende Mittel ist sehr gut konserviert und sehr gut verträglich.

EP 0 365 825 B1

**Beispiel 2: Duschbad**

| | |
|---|---|
| 0,2 g | Sorbinsäure |
| 0,1 g | Natriumformiat |
| 22,0 g | Laurylalkohol-diglykolethersulfat, Natriumsalz (70 prozentige, wäßrige Lösung) |
| 1,0 g | Ethylenglykoldistearat |
| 3,0 g | Kokosfettsäurediethanolamid |
| 1,0 g | Parfüm |
| 0,1 g | Phosphorsäure |
| 72,6 g | Wasser |
| 100,0 g | |

Der pH-Wert wird mit verdünnter Natronlauge auf 4,8 - 5,8 eingestellt. Das vorstehende Mittel ist sehr gut konserviert und hervorragend verträglich.

**Beispiel 3: Haarfestiger**

| | |
|---|---|
| 1,0 g | Sorbinsäure |
| 0,2 g | Natriumformiat |
| 1,0 g | Chitosan, niedrigviskos (zu 80 Prozent ent-acetyliert) |
| 7,0 g | Ethanol |
| 0,1 g | Zitronensäure |
| 90,7 g | Wasser |
| 100,0 g | |

sowohl zum Auflösen (Salzbildung) des Chitosans als auch zur Konservierung. Das vorstehende Mittel ist ausgezeichnet konserviert und sehr gut verträglich.

**Beispiel 4: Haarkurmittel - Rinse**

| | |
|---|---|
| 0,10 g | Sorbinsäure |
| 0,10 g | Natriumformiat |
| 1,00 g | Stearylalkohol |
| 1,00 g | Vaseline |
| 0,50 g | selbstemulgierendes Glycerinmonodistearat |
| 0,25 g | Cetyltrimethylammoniumchlorid |
| 0,10 g | Zitronensäure |
| 96,95 g | Wasser |
| 100,00 g | |

5

Der pH-Wert wird mit Zitronensäure auf 2,5 - 3,5 eingestellt. Das vorstehende Mittel ist ausgezeichnet konserviert und hervorragend verträglich.

Die Prozentangaben dieser Anmeldung stellen, soweit nichts anderes vermerkt, Gewichtsprozente dar.

## Patentansprüche

1. Wäßriges Haar- und Körperbehandlungsmittel mit einem Gehalt an 0,1 bis 50 Gewichtsprozent eines Tensids oder eines Tensidgemisches, dadurch gekennzeichnet, daß es eine Kombination aus

(A) einem physiologisch verträglichen Salz der Ameisensäure,

(B) Sorbinsäure und/oder deren physiologisch verträglichem Salz und

(C) einer oder mehrerer physiologisch verträglicher anorganischer oder gesättigter aliphatischer organischer Säuren

enthält.

2. Mittel nach Anspruch 1, dadurch gekennzeichnet, daß die Komponente (A) das Natriumformiat ist.

3. Mittel nach einem der Ansprüche 1 oder 2, dadurch gekennzeichnet, daß die Komponente (A) in einer Menge von 0,01 bis 2,0 Gewichtsprozent, vorzugsweise 0,1 bis 1,0 Gewichtsprozent, enthalten ist.

4. Mittel nach einem der Ansprüche 1 bis 3, dadurch gekennzeichnet, daß die Komponente (B) in einer Menge von 0,05 bis 2,0 Gewichtsprozent, vorzugsweise 0,1 bis 1,0 Gewichtsprozent, enthalten ist.

5. Mittel nach einem der Ansprüche 1 bis 4, dadurch gekennzeichnet, daß es einen pH-Wert von 2,0 bis 7,0, vorzugsweise von 3,0 bis 5,8, aufweist.

6. Mittel nach einem der Ansprüche 1 bis 5, dadurch gekennzeichnet, daß die Komponente (C) Zitronensäure oder Phosphorsäure ist.

7. Mittel nach einem der Ansprüche 1 bis 6, dadurch gekennzeichnet, daß die Komponente (C) in einer Menge von 0,1 bis 1,5 Gewichtsprozent, vorzugsweise 0,25 bis 0,5 Gewichtsprozent, enthalten ist.

8. Mittel nach einem der Ansprüchen 1 bis 7, dadurch gekennzeichnet, daß es als Lotion, als Gel, als Creme oder als Aerosol vorliegt.

9. Mittel nach einem der Ansprüche 1 bis 8, dadurch gekennzeichnet, daß es niedere aliphatische Alkohole, kationische, anionische, amphotere oder nichtionogene Tenside, natürliche, modifizierte natürliche oder synthetische Polymere, Verdicker, Pflegestoffe, Farbstoffe, Parfümöle, Komplexbildner oder Antioxidantien enthält.

10. Verwendung einer Kombination aus

(A) einem physiologisch verträglichen Salz der Ameisensäure,

(B) Sorbinsäure oder deren physiologisch verträglichem Salz und

(C) einer physiologisch verträglichen anorganischen oder aliphatischen organischen Säure zur Konservierung von Haar- und Körperreinigungsmitteln.

## Claims

1. Aqueous hair and body treatment composition containing 0.1 to 50 weight percent of surfactant or surfactant mixture, characterized in that it contains a combination of

(A) a physiolosically acceptable salt of formic acid

(B) sorbic acid and/or a physiologically acceptable salt thereof and

(C) one or a plurality of physiologically acceptable inorganic or saturated aliphatic organic acids.

2. Composition according to claim 1 characterized in that the component (A) is sodium formate.

3. Composition according to any one of claims 1 to 2, characterized in that it contains 0.01 to 2.0 weight percent, preferably 0.1 to 1.0 weight percent of component (A).

4. Composition according to any one of claims 1 to 3, characterized in that it contains 0.05 to 2.0 weight percent, preferably 0.1 to 1.0 weight percent of component (B).

5. Composition according to any one of claims 1 to 4 characterized in that it has a pH-value from 2.0 to 7.0 preferable from 3.0 to 5.8.

6. Composition according to any one of claims 1 to 5, characterized in that the component (C) is citric acid or phosphoric acid.

7. Composition according to any one of claims 1 to 6, characterized in that it contains 0.1 to 1.5 weight percent, preferably 0.25 to 0.5 weight percent of component (C).

8. Composition according to any one of claims 1 to 7 characterized in that it is available as lotion, sel cream or as an aerosol.

9. Composition according to any one of claims 1 to 8, characterized in that it contains lower aliphatic alcohols, cationic, anionic, amphoteric or nonionic surfactants, natural, modified natural or synthetic polymers, thickeners, care products, colouring agents, perfume oils, complex formers or antioxidants.

10. Use of a combination of

(A) a physiologically acceptable formic acid salt

(B) sorbic acid or a physiolosically acceptable salt thereof and

(C) a pysiologically acceptable inorsanic or aliphatic organic acid for the preservation of hair and body cleansing media.

**Revendications**

1. Produit aqueux de soins capillaires et corporels contenant de 0,1 à 50 pour cent en poids d'un agent tensioactif ou d'un mélange tensio-actif, caractérisé en ce qu'il contient une combinaison

A) d'un sel physiologiquement tolérable de l'acide formique,

B) d'acide sorbique et/ou de son sel physiologiquement tolérable et

C) d'un ou de plusieurs acides minéraux ou organiques aliphatiques saturés, physiologiquement tolérables.

2. Produit selon la revendication 1, caractérisé en ce que le composant A) est du formiate de sodium.

3. Produit selon la revendication 1 ou 2, caractérisé en ce que le composant A) est contenu à raison de 0,01 à 2 pour cent en poids, de préférence à raison de 0,1 à 1 pour cent en poids.

4. Produit selon l'une des revendications 1 à 3, caractérisé en ce que le composant B) est contenu à raison de 0,05 à 2 pour cent en poids, de préférence à raison de 0,1 à 1 pour cent en poids.

5. Produit selon l'une des revendications 1 à 4, caractérisé en ce qu'il présente un pH compris entre 2 et 7, de préférence entre 3 et 5,8.

6. Produit selon l'une des revendications 1 à 5, caractérisé en ce que le composant C) est de l'acide de citrique ou de l'acide phosphorique.

7. Produit selon l'une des revendications 1 à 6, caractérisé en ce que le composant C) est contenu à raison de 0,1 à 1,5 pour cent en poids, de préférence à raison de 0,25 à 0,5 pour cent en poids.

8. Produit selon l'une des revendications 1 à 7, caractérisé en ce qu'il se présente sous la forme d'une lotion, d'un gel, d'une crème ou d'un aérosol.

9. Produit selon l'une des revendications 1 à 8, caractérisé en ce qu'il contient des alcools aliphatiques inférieurs, des agents tensio-actifs cationiques, anioniques, amphotères ou non - ionogènes, des polymères naturels, naturels modifiés ou synthétiques, des épaississants, des produits de cure, des colorants, des essences de parfum, des complexants ou des antioxydants.

10. Utilisation d'une combinaison

A) d'un sel physiologiquement tolérable de l'acide formique,

B) d'acide sorbique et/ou de son sel physiologiquement tolérable et

C) d'un ou de plusieurs acides minéraux ou organiques aliphatiques saturés, physiologiquement tolérables, pour la conservation de produits de soins capillaires et corporels.